(19) Europäisches Patentamt
European Patent Office
Office européen des brevets

(11) Veröffentlichungsnummer: **0 011 802**
**B1**

(12) # EUROPÄISCHE PATENTSCHRIFT

(45) Veröffentlichungstag der Patentschrift: **15.09.82**

(21) Anmeldenummer: **79104578.4**

(22) Anmeldetag: **19.11.79**

(51) Int. Cl.³: **C 07 C 103/34,** C 07 C 103/365, C 07 C 103/37, C 07 C 103/375, C 07 C 103/38, C 07 C 103/44, C 07 C 121/43, C 07 D 213/75, C 07 D 239/42, A 01 N 39/02 // (C07C103/34, 79/35)

(54) **Neue Phenoxy-phenoxypropionsäureamide, Verfahren zu ihrer Herstellung, sie enthaltende herbizide Mittel und ihre Verwendung zur Bekämpfung von Schadpflanzen.**

(30) Priorität: **24.11.78 DE 2850902**

(43) Veröffentlichungstag der Anmeldung: **11.06.80 Patentblatt 80/12**

(45) Bekanntmachung des Hinweises auf die Patenterteilung: **15.09.82 Patentblatt 82/37**

(84) Benannte Vertragsstaaten: **AT BE CH DE FR GB IT NL**

(56) Entgegenhaltungen:
**EP-A-0 000 474**
**EP-A-0 003 295**
**DE-A-2 632 581**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt/Main 80 (DE)**

(72) Erfinder: **Schönowsky, Hubert, Dr., Siegweg 1, D-6074 Rödermark (DE)**
Erfinder: **Bieringer, Hermann, Dr., Eichenweg 26, D-6239 Eppstein/Taunus (DE)**
Erfinder: **Köcher, Helmut, Dr., Kleiststrasse 9, D-6238 Hofheim am Taunus (DE)**

**EP 0 011 802 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue Phenoxy-phenoxypropionsäureamide, Verfahren zu ihrer Herstellung, sie enthaltende herbizide Mittel und ihre Verwendung zur Bekämpfung von Schadpflanzen

Gegenstand vorliegender Erfindung sind neue Phenoxy-phenoxy-propionsäureamide der allgemeinen Formel I

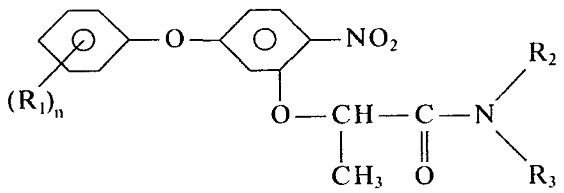

in der

$R_1$ = Halogen, $NO_2$, CN und/oder $CF_3$;
n = 1 oder 2;
$R_2$ = Wasserstoff oder $(C_1-C_4)$Alkyl;
$R_3$ = $(C_3-C_6)$Alkenyl, $(C_3-C_6)$Alkinyl, Hydroxy-$(C_1-C_4)$alkyl, $(C_1-C_4)$Alkoxy-$(C_1-C_4)$alkyl, Di-$(C_1-C_4)$alkylamino-$(C_1-C_4)$alkyl, Cyano-$(C_1-C_4)$alkyl, $(C_1-C_4)$Alkylthio-$(C_1-C_4)$alkyl, $(C_5-C_6)$Cycloalkyl, Methyl-cyclohexyl, Phenyl-$(C_1-C_4)$alkyl, Pyridin- oder Pyrimidinrest bedeutet oder
$R_2$ und $R_3$ zusammen mit dem benachbarten N-Atom einen 4–7gliedrigen Ring bilden, in dem bis zu zwei C-Atome durch N und/oder O ersetzt sein können.

Die erfindungsgemäßen Verbindungen der allgemeinen Formel I lassen sich dadurch herstellen, daß man Phenoxy-phenoxypropionsäurehalogenide der allgemeinen Formel II (Methode A) oder Phenoxy-phenoxy-propionsäureester der allgemeinen Formel III (Methode B) mit entsprechenden Aminen der allgemeinen Formel IV umsetzt:

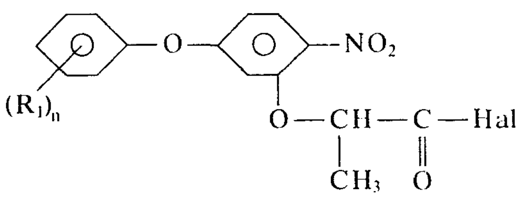

(II)

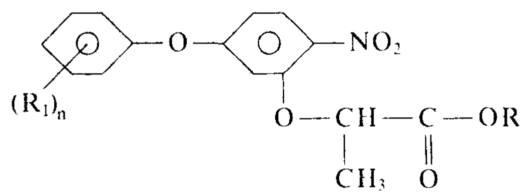

(III)

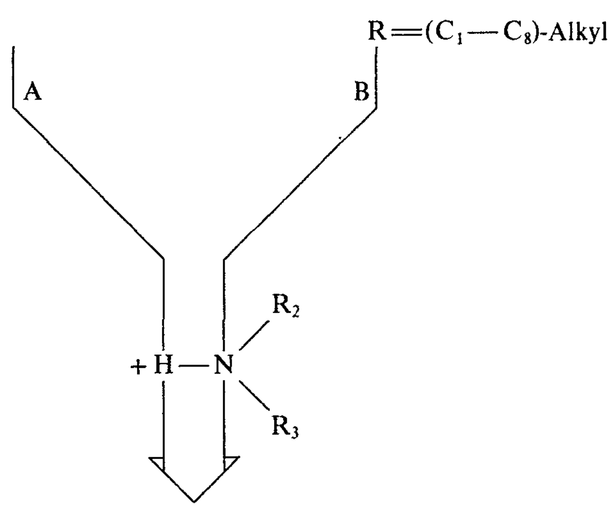


(IV)

(I)

oder indem man substituierte 3-Hydroxy-4-nitro-diphenyläther mit Halogenpropionsäureamiden reagieren läßt (Methode C):

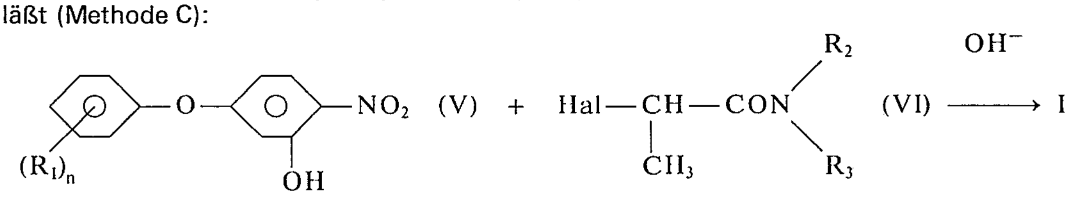


A) Die Umsetzung der Säurehalogenide (II) mit Aminen wird vorteilhaft in einem inerten organischen Lösungsmittel wie z. B. Methylethylketon. Dimethoxiethan, Benzol, Toluol, Xylol u. a. vorgenommen. Man arbeitet in Gegenwart einer tert. organischen Base wie z. B. Triethylamin oder in Gegenwart einer alkalischen Verbindung wie z. B. MgO oder $K_2CO_3$ zur Bindung des freiwerdenden Halogenwasserstoffs bei Temperaturen zwischen 25 und 80°.
Nach beendeter Reaktion wird das gebildete halogenwasserstoffsaure Salz durch Filtration oder durch Behandeln mit Wasser abgetrennt, und — gegebenenfalls nach anschließender Entfernung des organischen Lösungsmittels — das gebildete Amid isoliert.

B) Die Phenoxy-phenoxy-propionsäureester (III) werden durch Umsetzung in einem Überschuß des jeweiligen Amins IV in die entsprechenden Amide (I) überführt. Man arbeitet bevorzugt in wäßriger Lösung bei Temperaturen von 40 – 80° C.

C) Die direkte Umsetzung von V mit VI wird üblicherweise ebenfalls in einem der unter A) beschriebenen Lösungsmittel und in Gegenwart von Basen wie $K_2CO_3$ oder Triäthylamin zur Bindung des freiwerdenden Halogenwasserstoffs durchgeführt; alternativ kann man auch von den durch Umsetzung von V mit Alkalien erhaltenen Phenolaten ausgehen. Die Reaktionstemperaturen liegen hier zwischen 40° und dem Siedepunkt des Lösungsmittels.
Die für die Umsetzungen jeweils benötigten Ausgangsstoffe der Formalen II, III bzw. V werden nach literaturbekannten Methoden hergestellt und sind z. B. in der DE-OS 2 632 581 beschrieben.

Die so erhaltenen Verbindungen (I) können nach üblichen Verfahren, z. B. Umkristallisieren, gereinigt werden.

Die erfindungsgemäßen Verbindungen zeichnen sich gegenüber bereits bekannten Verbindungen ähnlicher Struktur (vgl. DE-OS 2 632 581, US-PS 3 652 645) durch bessere herbizide Wirksamkeit und besondere Selekivität aus. So zeigen sie vor allem im Vorauflauf-Verfahren gute herbizide Wirkung gegen eine Anzahl von dikotylen Unkräutern und Ungräsern, die in den verschiedensten landwirtschaftlichen Anbauregionen der Erde wirtschaftlich bedeutende Probleme darstellen. Insbesondere werden schwer zu bekämpfende Vertreter wie z. B. Klettenlabkraut (Galium) — eine wichtige Schadpflanze in europäischen wie amerikanischen Anbaugebieten — neben weiteren Unkrautarten gut bekämpft.

Die erfindungsgemäßen Verbindungen (I) zeichnen sich durch besondere Verträglichkeit in zahlreichen Kulturen wie z. B. Getreide, Reis, Mais, Soja, Baumwolle, Raps usw. aus. In den genannten Kulturen werden noch bei Konzentrationen von 2,4 kg/ha keine oder nur geringfügige Schäden beobachtet.

Die Verbindungen können daher zur Herstellung von herbiziden Mitteln verwendet werden, in denen die Wirkstoffe der Formel (I) zu 2–95% enthalten sind. Diese Mittel können als emulgierbare Konzentrate, Spritzpulver, versprühbarer Lösungen, Stäubemittel und Granulate in den üblichen Zubereitungsformen angewendet werden.

Spritzpulver sind in Wasser gleichmäßig dispergierbare Präparate, die neben dem Wirkstoff außer einem Verdünnungs- oder Inertstoff noch Netzmittel, z. B. polyoxethylierte Alkylphenole, polyoxethylierte Fettalkohole, Alkyl- oder Alkylphenyl-sulfonate und Dispergiermittel, z. B. ligninsulfonsaures Natrium, 2,2′-dinaphthylmethan-6,6′-disulfonsaures Natrium, dibutylnaphthalinsulfonsaures Natrium oder auch oleylmethyltaurinsulfonsaures Natrium enthalten.

Emulgierbare Konzentrate werden durch Auflösen des Wirkstoffes in einem organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwandt werden:

Alkylarylsulfonsaure Calziumsalze wie Ca-dodecylbenzolsulfonat, oder
nichtionische Emulgatoren wie Fettsäurepolyglykolester,
Alkylarylpolyglykolether, Fettalkoholpolyglykolether,
Propylenoxid-Ethylenoxid-Kondensationsprodukte,
Fettalkohol-Propylenoxid-Ethylenoxid-Kondensationsprodukte,
Alkylpolyether, Sorbitanfettsäureester, Polyoxethylen-sorbitan-fettsäureester oder
Polyoxethylen-sorbitester.

Stäubemittel erhält man durch Vermahlen des Wirkstoffes mit verteilten, festen Stoffen, z. B.

Talkum, natürlichen Tonen, wie Kaolin, Bentonit, Pyrophillit oder Diatomeenerde.

Versprühbare Lösungen, wie sie vielfach in Sprühdosen gehandelt werden, enthalten den Wirkstoff in einem organischen Lösungsmittel gelöst neben einem geeigneten, inerten Treibmittel.

Granulate können entweder durch Verdüsen des Wirkstoffes auf adsorptionsfähiges, granuliertes Inertmaterial hergestellt werden oder durch Aufbringen von Wirkstoffkonzentrationen mittels Klebemitteln, z. B. Polyvinylalkohol, polyacrylsaurem Natrium oder auch Mineralölen auf die Oberfläche von Trägerstoffen, wie Sand, Kaolinite, oder von granuliertem Inertmaterial. Auch können geeignete Wirkstoffe in der für die Herstellung von Düngemittelgranalien üblichen Weise — gewünschtenfalls in Mischung mit Düngemitteln — hergestellt werden.

Bei herbiziden Mitteln können die Konzentrationen der Wirkstoffe in den handelsüblichen Formulierungen verschieden sein. In Spritzpulvern variiert die Wirkstoffkonzentration, z. B. zwischen etwa 10 und 80%, der Rest besteht aus den oben angegebenen Formulierungszusätzen. Bei emulgierbaren Konzentraten ist die Wirkstoffkonzentration etwa 10 bis 80%. Staubförmige Formulierungen enthalten meistens 5–20% an Wirkstoff, versprühbare Lösungen etwa 2–20%. Bei Granulaten hängt der Wirkstoffgehalt z. T. davon ab, ob die wirksame Verbindung flüssig oder fest vorliegt und welche Granulierhilfsmittel, Füllstoffe usw. verwendet werden.

Zur Anwendung werden die handelsüblichen Konzentrate, gegebenenfalls in üblicher Weise verdünnt, z. B. bei Spritzpulvern und emulgierbaren Konzentraten mittels Wasser. Staubförmige und granulierte Zubereitungen sowie versprühbare Lösungen werden vor der Anwendung nicht mehr mit weiteren inerten Stoffen verdünnt. Mit den äußeren Bedingungen wie Temperatur, Feuchtigkeit u. a. variiert die erforderliche Aufwandmenge. Sie kann innerhalb weiter Grenzen schwanken, z. B. zwischen 0,1 und 10,0 kg Aktivsubstanz/ha, vorzugsweise liegt sie jedoch zwischen 0,3 und 5 kg/ha.

Die erfindungsgemäßen Wirkstoffe können mit anderen Herbiziden und Bodeninsektiziden kombiniert werden.

Eine andere Anwendungsform der vorliegenden Wirkstoffe besteht in ihrer Mischung mit Düngemitteln, wodurch düngende und zugleich herbizide Mittel erhalten werden.

## Herstellungsbeispiele

### Allgemeine Vorschriften für Phenoxy-phenoxy-propionsäureamide der allgemeinen Formel I

### Methode A

Zu 0,1 Mol Säurechlorid, gelöst in 50 ml Toluol, werden 0,1 Mol Triethylamin zugesetzt. Bei 25–40°C werden dann 0,1 Mol eines Amins zugetropft. Man läßt 1 Std. nachreagieren und setzt dann Wasser zu. Die Toluolphase wird abgetrennt, mit Wasser gewaschen und über Natriumsulfat getrocknet. Nach Abdestillieren des Toluols wird das Amid isoliert und gereinigt.

### Methode B

0,1 Mol Phenoxi-phenoxi-propionsäureester werden bei 40°C in 0,2 Mol einer 50%igen wäßrigen Aminlösung eingetragen. Nach 2 Std. Rühren bei 50°C wird abgesaugt, mit Wasser gewaschen und der kristalline Rückstand getrocknet.

Beispiele für die so erhaltenen Verbindungen sind in folgender Tabelle aufgeführt:

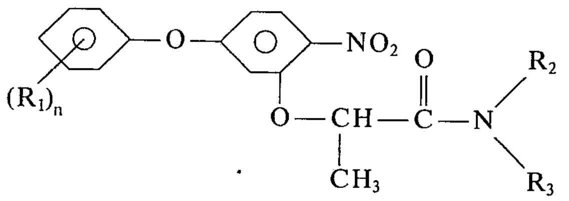

| Beispiel Nr. | $(R_1)_n$ | $R_2$ | $R_3$ | Fp. [°C] (bzw. $n_D$) | Methode |
|---|---|---|---|---|---|
| 1 | 2,4-Cl | H | $-(CH_2)_2-OCH_3$ | 133—135 | B |
| 2 | 4-Br | H | $-CH_2-CH=CH_2$ | | A |
| 3 | 2,Cl, 4-Br | H | $-CH_2-CH=CH_2$ | | B |
| 4 | 2,4-Br | H | $-(CH_2)_2-OCH_3$ | | A |
| 5 | 2,4-Cl | H | $-C(CH_3)_2-CN$ | 160—161 | A |
| 6 | 2,4-Cl | H | $-CH_2-CH=CH_2$ | 125—127 | A |
| 7 | 2,4-Cl | H | $-(CH_2)_2-OH$ | Öl | A |
| 8 | 2,4-Cl | H | $-C(CH_3)_2-CH_2OH$ | 105—108 | B |
| 9 | 2,4-Cl | H | $-CH_2-$ 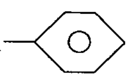 | 157—159 | A |
| 10 | 2,4-Cl | H | $-C(CH_3)_2-$ 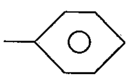 | 113—116 | A |
| 11 | 2-Cl, 4-$CF_3$ | H | $-CH_2-CH=CH_2$ | 123—126 | A |
| 12 | 2-Cl, 4-$CF_3$ | H | $-(CH_2)_2-OCH_3$ | 102—105 | B |
| 13 | 2,4-Cl | H | $-(CH_2)_2-SCH_3$ | 109—111 | B |
| 14 | 2,4-Cl | H | $-(CH_2)_3-SCH_3$ | 82 | B |
| 15 | 2,4-Cl | H | $-(CH_2)_3-N(CH_3)_2$ | 74— 77 | B |
| 16 | 2,4-Cl | $CH_3$ | $-CH(CH_3)-C\equiv CH$ | 98—101 | A |
| 17 | 2,4-Cl | $CH_3$ | $-(CH_2)_2-CN$ | Öl | A |
| 18 | 2,4-Cl | H | $-(CH_2)_3-OCH(CH_3)_2$ | 84— 86 | A |
| 19 | 2,4-Cl | H | 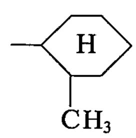 | 167—170 | A |
| 20 | 2,4-Cl | H | 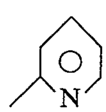 | 165—168 | A |
| 21 | 2,4-Cl | H | 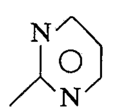 | | |
| 22 | 2,4-Cl | | $-CH_2CH_2-O-CH_2CH_2-$ | 91— 93 | B |
| 23 | 2,4-Cl | | $-CH_2CH_2CH_2CH_2CH_2-$ | 96— 99 | B |

Fortsetzung

| Beispiel Nr. | $(R_1)_n$ | $R_2$ | $R_3$ | Fp. [°C] (bzw. $n_D$) | Methode |
|---|---|---|---|---|---|
| 24 | 2,4-Cl | | $-CH_2CH_2\underset{\displaystyle CH_3}{\underset{\vert}{C}}HCH_2CH_2-$ | 113—115 | A |
| 25 | 2,4-Cl | | $-CH_2CH_2CH_2CH_2-$ | 63— 66 | A |
| 26 | 2-Cl, 4-$CF_3$ | H | $-(CH_2)_2-SCH_3$ | 95— 98 | A |
| 27 | 2-Cl, 4-$CF_3$ | H | $-(CH_2)_3-N(CH_3)_2$ | 81— 83 | A |
| 28 | 2-Cl, 4-$CF_3$ | H | $-(CH_2)_2-OH$ | 98—101 | A |
| 29 | 4-$CF_3$ | H | $-(CH_2)_2-OCH_3$ | 88— 91 | A |
| 30 | 4-$CF_3$ | H | $-CH_2-CH=CH_2$ | 128—130 | A |
| 31 | 4-$CF_3$ | H | $-(CH_2)_2-SCH_3$ | 83— 85 | A |
| 32 | 4-$CF_3$ | H | $-(CH_2)_3-N(CH_3)_2$ | 63— 65 | A |
| 33 | 4-$CF_3$ | H | $-(CH_2)_2-OH$ | 95— 98 | A |

## Formulierungsbeispiele

### Beispiel A

Ein in Wasser leicht dispergierbares benetzbares Pulver wird erhalten, indem man

| | |
|---|---|
| 25 Gewichtsteile | 2-[3-(2',4'-Dichlorphenoxy)-6-nitrophenoxy]-propionsäure-methoxiethylamid als Wirkstoff, |
| 64 Gewichtsteile | kaolinhaltigen Quarz als Inertstoff, |
| 10 Gewichtsteile | ligninsulfonsaures Kalium und |
| 1 Gewichtsteil | oleylmethyltaurinsaures Natrium als Netz- und Dispergiermittel |

mischt und in einer Stiftmühle mahlt.

### Beispiel B

Ein Stäubemittel, das sich zur Anwendung als Unkrautvertilgungsmittel gut eignet, wird erhalten, indem man

| | |
|---|---|
| 10 Gewichtsteile | 2-[3-(2',4'-Dichlorphenoxy)-6-nitrophenoxy]-propionsäure-methoxi-ethylamid als Wirkstoff und |
| 90 Gewichtsteile | Talkum als Inertstoff |

mischt und in einer Schlagmühle zerkleinert.

### Beispiel C

Ein emulgierbares Konzentrat besteht aus

| | |
|---|---|
| 15 Gewichtsteilen | 2-[3-(2',4'-Dichlorphenoxi)-6-nitrophenoxi]-propionsäure-methoxiethylamid, |
| 75 Gewichtsteilen | Cyclohexanon als Lösungsmittel und |
| 10 Gewichtsteilen | oxethyliertes Nonylphenol (10 AeO) als Emulgator. |

Beispiel D

Ein Granulat besteht z. B. aus etwa

2—15 Gewichtsteilen 2-[3-(2',4'-Dichlorphenoxi)-6-nitro-phenoxi]-propionsäure-methoxiethylamid

und inerten Granulatträgermaterialien, wie z. B. Attapulgit, Bimsgranulat und Quarzsand.

## Biologische Beispiele

Erfindungsgemäße Verbindungen in Form wäßriger Dispersionen von Spritzpulverkonzentraten wurden nach dem Aussäen von Unkräutern auf Blumentöpfe gesprüht und anschließend hinsichtlich ihrer herbiziden Effektivität beobachtet und visuell bonitiert. Die Bonitierung erfolgte als prozentuale Schätzung der herbiziden Schadwirkung (Vorauflaufbehandlung).

Die Ergebnisse in Tabelle I zeigen, daß die Substanzen sehr gute herbizide Eigenschaften haben und zahlreiche Unkräuter gut bekämpfen.

Ebenso wurden verschiedene Unkräuter in Töpfe ausgesät und im Gewächshaus ca. 3 Wochen bis zur Größe von 15 bis 25 cm herangezogen. Anschließend wurden die Pflanzen im Nachauflaufverfahren mit den erfindungsgemäßen Verbindungen behandelt. Die visuelle Bonitur, ca. 4 Wochen nach Applikation durchgeführt, zeigte, daß die Unkräuter von den Verbindungen gut bekämpft wurden und dem Vergleichspräparat Bifenox [5-(2,4-Dichlorphenoxy)-2-nitrobenzoesäuremethylester] insbesondere im Nachauflauf überlegen sind (s. Tab. I).

Ferner wurden Kulturpflanzen in Töpfe ausgesät und vor dem Auflaufen mit den erfindungsgemäßen Verbindungen behandelt. Es zeigte sich, daß selbst hohe Dosierungen von 2,4 kg AS/ha (AS = Aktivsubstanz) keine oder nur geringfügige Schäden an den Kulturpflanzen verursachen. Die Bonitur erfolgte ca. 4 Wochen nach Applikation der Verbindungen. Die Ergebnisse sind in Tabelle II aufgeführt. Die Versuchstöpfe wurden unter Gewächshausbedingungen gehalten. Die Ergebnisse belegen, daß erfindungsgemäße Verbindungen von verschiedenen Kulturpflanzen selbst bei hohen Dosen von 2,4 kg sehr gut toleriert werden oder nur geringe Schäden verursachen. Somit können diese erfindungsgemäßen Verbindungen zur selekt. Unkrautbekämpfung in landwirtschaftlichen Kulturen eingesetzt werden.

Tabelle I

Herbizide Wirksamkeit der Verbindungen in Prozent gegen verschiedene Unkräuter

| Beispiel Nr. | Vorauflaufverfahren | | | | | | Nachauflaufverfahren | | |
|---|---|---|---|---|---|---|---|---|---|
| | kg AS/ha | SAL | ECG | AMR | MAT | CRS | MAT | AMR | CRS |
| 6 | 2,4 | 100 | 65 | 100 | 90 | 75 | — | — | — |
| | 0,6 | 90 | — | 100 | — | — | — | — | — |
| 1 | 2,4 | 100 | 100 | 100 | 96 | 95 | — | — | — |
| | 0,6 | 100 | 95 | 100 | 95 | 85 | — | — | — |
| 7 | 2,4 | 95 | 75 | 100 | 100 | 100 | 65 | — | — |
| | 0,6 | 85 | — | 100 | 98 | 100 | 65 | — | — |
| 11 | 2,4 | 100 | 95 | 100 | 100 | 100 | 90 | — | — |
| 12 | 2,4 | 100 | 100 | 100 | 100 | 100 | 100 | — | — |
| | 0,6 | 100 | 100 | 100 | 100 | 100 | 100 | — | — |
| 22 | 2,4 | 100 | 100 | 100 | 80 | — | — | — | — |
| | 0,6 | 90 | 60 | 100 | 80 | — | — | — | — |
| 8 | 2,4 | 75 | 75 | 100 | 90 | 75 | — | 100 | — |
| 9 | 2,4 | 65 | 75 | 100 | 75 | 65 | — | 95 | — |
| 13 | 2,4 | 65 | — | 100 | 98 | 85 | 90 | 95 | 80 |
| | 0,6 | 35 | — | 100 | 95 | 35 | 85 | 70 | 40 |

Fortsetzung

| Beispiel Nr. | Vorauflaufverfahren kg AS/ha | SAL | ECG | AMR | MAT | CRS | Nachauflauf-verfahren MAT | AMR | CRS |
|---|---|---|---|---|---|---|---|---|---|
| 14 | 2,4 | 65 | – | 100 | – | 65 | – | 80 | – |
| 15 | 2,4 | – | – | 100 | 75 | – | – | 95 | – |
| 16 | 2,4 | – | – | – | – | – | 100 | 100 | 98 |
| | 0,6 | – | – | – | – | – | 95 | 100 | 80 |
| 18 | 2,4 | 100 | 85 | 100 | 100 | 100 | 98 | 100 | 100 |
| | 0,6 | 95 | 65 | 100 | 85 | 95 | 90 | 100 | 98 |
| 20 | 2,4 | – | – | – | – | – | 95 | 100 | 98 |
| | 0,6 | – | – | – | – | – | 90 | 100 | 80 |
| 23 | 2,4 | – | – | 100 | – | – | 100 | 100 | 100 |
| | 0,6 | – | – | 95 | – | – | 85 | 98 | 60 |
| 24 | 2,4 | – | – | 100 | – | – | 95 | 100 | 98 |
| | 0,6 | – | – | – | – | – | 80 | 100 | 80 |
| 25 | 2,4 | 98 | – | 100 | 90 | 85 | 95 | 100 | 100 |
| | 0,6 | 65 | – | 100 | 35 | 35 | 90 | 100 | 80 |
| 26 | 2,4 | 100 | 98 | 100 | 100 | 100 | 100 | 100 | 100 |
| | 0,6 | 100 | 60 | 100 | 100 | 100 | 100 | 100 | 100 |
| 27 | 2,4 | 100 | 90 | 100 | 100 | 100 | 100 | 100 | 100 |
| | 0,6 | 98 | 20 | 100 | 100 | 100 | 100 | 100 | 100 |
| 28 | 2,4 | 100 | 100 | 100 | 100 | 100 | 100 | 100 | 100 |
| | 0,6 | 100 | 100 | 100 | 100 | 100 | 100 | 100 | 100 |
| 29 | 2,4 | 100 | 100 | 100 | 100 | 80 | – | – | – |
| | 0,6 | 100 | 100 | 100 | 100 | 60 | – | – | – |
| 30 | 2,4 | 100 | 95 | 100 | – | – | 90 | 100 | 95 |
| | 0,6 | 100 | 50 | 100 | – | – | 70 | 98 | 70 |
| 31 | 2,4 | 100 | 80 | 100 | 100 | 90 | – | – | – |
| | 0,6 | 95 | 30 | 100 | 80 | 40 | – | – | – |
| 32 | 2,4 | – | – | 100 | – | – | – | – | – |
| | 0,6 | – | – | 100 | – | – | – | – | – |
| 33 | 2,4 | 100 | 100 | 100 | 100 | 98 | – | – | – |
| | 0,6 | 100 | 98 | 100 | 100 | 98 | – | – | – |
| Vergleichsmittel | 2,4 | 85 | 90 | 100 | 100 | 60 | 35 | 70 | 20 |
| (Bifenox) | 0,6 | 65 | 50 | 95 | 100 | 35 | 0 | – | – |

SAL = Setaria.
ECG = Echinochloa.
AMR= Amaranthus.
MAT = Matricaria.
CRS = Chrysanthemum.

Tabelle II

Verträglichkeit an Kulturpflanzen (Schadwirkung in %) im Vorauflaufverfahren

| Beispiel Nr. | kg AS/ha | Sojabohne | Baumwolle | Raps | Sorghum |
|---|---|---|---|---|---|
| 1 | 2,4 | 0 | 0 | 5 | 0 |
| 7 | 2,4 | 6 | – | 0 | 0 |
| 22 | 2,4 | 0 | 0 | 0 | 0 |

**Patentansprüche für die Vertragsstaaten: BE, CH, DE, FR, GB, IT, NL**

1. Phenoxy-phenoxy-propionsäureamide der allgemeinen Formel I

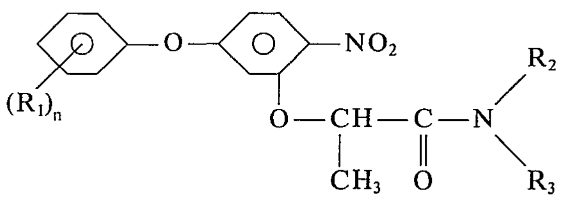

(I)

worin

$R_1$ = Halogen, $NO_2$, CN und/oder $CF_3$;
n = 1 oder 2;
$R_2$ = Wasserstoff oder $(C_1-C_4)$Alkyl;
$R_3$ = $(C_3-C_6)$Alkenyl, $(C_3-C_6)$Alkinyl, Hydroxy-$(C_1-C_4)$alkyl, $(C_1-C_4)$Alkoxy-$(C_1-C_4)$alkyl, Di-$(C_1-C_4)$alkylamino-$(C_1-C_4)$alkyl, Cyano-$(C_1-C_4)$alkyl, $(C_1-C_4)$Alkylthio-$(C_1-C_4)$alkyl, $(C_5-C_6)$Cycloalkyl, Methyl-cyclohexyl, Phenyl-$(C_1-C_4)$alkyl, einen Pyridin- oder Pyrimidinrest bedeuten oder
$R_2$ und $R_3$ zusammen mit dem benachbarten N-Atom einen 4–7gliedrigen Ring bilden, in dem bis zu zwei C-Atome durch N und/oder O ersetzt sein können.

2. Verfahren zur Herstellung von Verbindungen der allgemeinen Formel I, dadurch gekennzeichnet, daß man

a) Phenoxy-phenoxy-propionsäurehalogenide der Formel

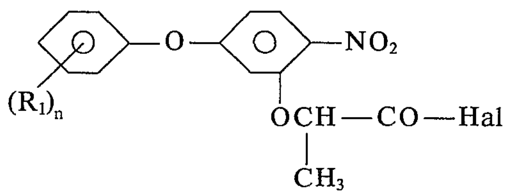

(II)

oder Phenoxy-phenoxy-propionsäureester der Formel

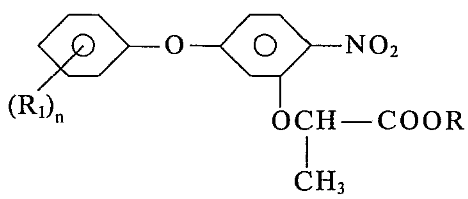

(III)

worin R $(C_1-C_8)$-Alkyl bedeutet, mit Aminen der Formel

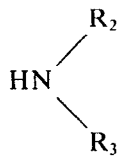

(IV)

oder

b) 3-Hydroxy-4-nitro-diphenyläther der Formel

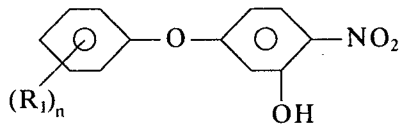

(V)

bzw. entsprechende Phenolate, mit Halogenpropionsäureamiden der Formel

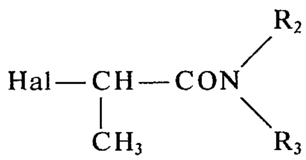

(VI)

worin »Hal« Chlor oder Brom bedeutet, gegebenenfalls in Gegenwart säurebindender Mittel umsetzt.

3. Herbizide Mittel, gekennzeichnet durch einen Gehalt an einer Verbindung der Formel I.
4. Verwendung von Verbindungen der Formel I zur Bekämpfung von Schadpflanzen.
5. 2-[3-(2',4'-Dichlorphenoxy)-6-nitrophenoxy]-propionsäure-hydroxyethylamid.
6. 2-[3-(2'-Chlor-4'-trifluormethylphenoxy)-6-nitrophenoxy]-propionsäure-methoxyethylamid.
7. 2-[3-(2'-Chlor-4'-trifluormethylphenoxy)-6-nitrophenoxy]-propionsäure-methylthioethylamid.
8. 2-[3-(2'-Chlor-4'-trifluormethylphenoxy)-6-nitrophenoxy]-propionsäure-hydroxyethylamid.

**Patentansprüche für den Vertragsstaat: AT**

1. Verfahren zur Herstellung von Phenoxy-phenoxy-propionsäureamiden der allgemeinen Formel I

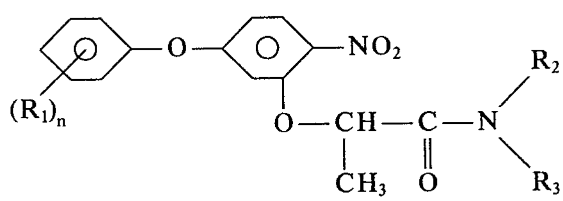

(I)

worin

$R_1$ = Halogen, $NO_2$, CN und/oder $CF_3$;
n = 1 oder 2;
$R_2$ = Wasserstoff oder $(C_1-C_4)$Alkyl;
$R_3$ = $(C_3-C_6)$Alkenyl, $(C_3-C_6)$Alkinyl, Hydroxy-$(C_1-C_4)$alkyl, $(C_1-C_4)$Alkoxy-$(C_1-C_4)$alkyl, Di-$(C_1-C_4)$alkylamino-$(C_1-C_4)$alkyl, Cyano-$(C_1-C_4)$alkyl, $(C_1-C_4)$Alkylthio-$(C_1-C_4)$alkyl, $(C_5-C_6)$Cycloalkyl, Methyl-cyclohexyl, Phenyl-$(C_1-C_4)$alkyl, einen Pyridin- oder Pyrimidinrest bedeuten oder
$R_2$ und $R_3$ zusammen mit dem benachbarten N-Atom einen 4–7gliedrigen Ring bilden, in dem bis zu zwei C-Atome durch N und/oder O ersetzt sein können,

dadurch gekennzeichnet, daß man

a) Phenoxy-phenoxy-propionsäurehalogenide der Formel

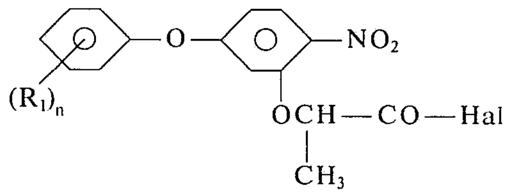

(II)

oder Phenoxy-phenoxy-propionsäureester der Formel

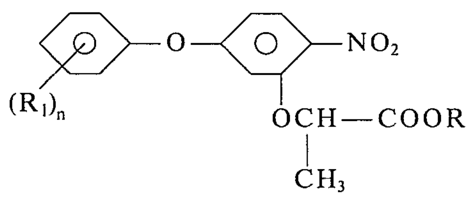

(III)

worin R ($C_1$—$C_8$)-Alkyl bedeutet, mit Aminen der Formel

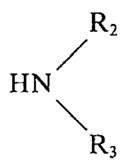

(IV)

oder

b) 3-Hydroxy-4-nitro-diphenyläther der Formel

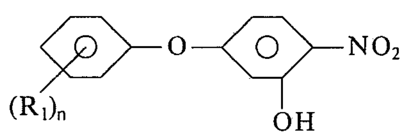

(V)

bzw. entsprechende Phenolate, mit Halogenpropionsäureamiden der Formel

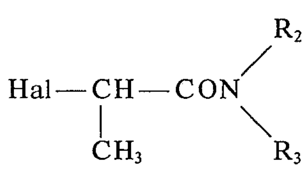

(VI)

worin »Hal« Chlor oder Brom bedeutet, gegebenenfalls in Gegenwart säurebindender Mittel umsetzt.

2. Herbizide Mittel, gekennzeichnet durch einen Gehalt an einer Verbindung der Formel I.
3. Verwendung von Verbindungen der Formel I zur Bekämpfung von Schadpflanzen.

**Claims for the Contracting states: BE, CH, DE, FR, GB, IT, NL**

1. Phenoxy-phenoxy-propionic acid amides of the general formula I

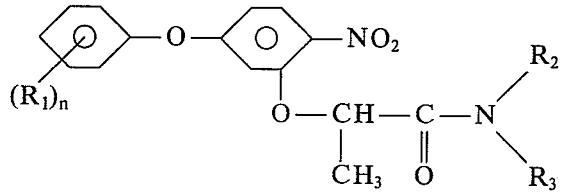

(I)

in which

$R_1$ is halogen, $NO_2$, CN and/or $CF_3$;
n is 1 or 2;
$R_2$ is hydrogen or $(C_1-C_4)$alkyl;
$R_3$ is $(C_3-C_6)$alkenyl, $(C_3-C_6)$alkinyl, hydroxy-$(C_1-C_4)$alkyl, $(C_1-C_4)$alkoxy-$(C_1-C_4)$alkyl, di-$(C_1-C_4)$alkylamino-$(C_1-C_4)$alkyl, cyano-$(C_1-C_4)$alkyl, $(C_1-C_4)$alkylthio-$(C_1-C_4)$alkyl, $(C_5-C_6)$cycloalkyl, methylcyclohexyl, phenyl-$(C_1-C_4)$alkyl, pyridyl or pyrimidyl; or
$R_2$ and $R_3$ together with the adjacent N-atom form a 4 to 7-membered ring in which up to 2 carbon atoms can be replaced by N and/or O.

2. Process for the manufacture of compounds of the general formula I which comprises reacting

a) phenoxy-phenoxy-propionic acid halides of the formula

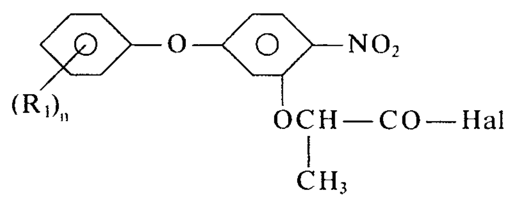

(II)

or phenoxy-phenoxy-propionic acid esters of the formula

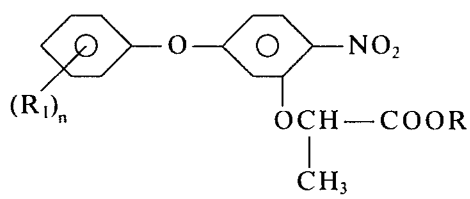

(III)

in which R is $(C_1-C_8)$alkyl, with amines of the formula

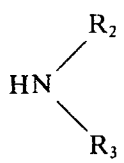

(IV)

or

b) reacting 3-hydroxy-4-nitro-diphenyl ethers of the formula

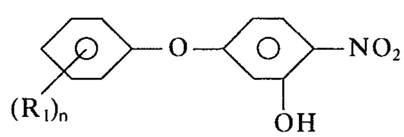

(V)

or correspondings phenolates, with halo-propionic acid amides of the formula

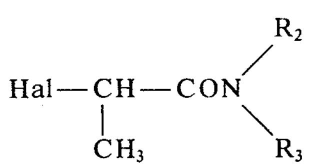

(VI)

in which Hal is chlorine or bromine, optionally in the presence of acid-binding agents.

3. Herbicidal agents, characterized by a content of a compound of the formula I.
4. The use of compounds of formula I for the combatting of weeds.
5. 2-[3-(2',4'-Dichlorphenoxy)-6-nitrophenoxy]-propionic acid hydroxyethyl amide.
6. 2-[3-(2'-Chloro-4'-trifluoromethylphenoxy)-6-nitrophenoxy]-propionic acid methoxyethyl amide.

7. 2-[3-(2'-Chloro-4'-trifluoromethylphenoxy)-6-nitrophenoxy]-propionic acid methylthioethyl amide.

8. 2-[3-(2'-Chloro-4'-trifluoromethylphenoxy)-6-nitrophenoxy]-propionic acid hydroxyethyl amide.

**Claims for the Contracting state: AT**

1. Process for the preparation of phenoxy-phenoxy-propionic acid amides of the general formula I

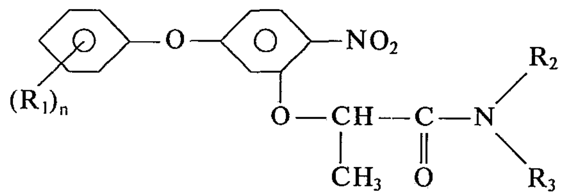

(I)

in which

$R_1$ is halogen, $NO_2$, CN and/or $CF_3$;
n is 1 or 2;
$R_2$ is hydrogen or $(C_1-C_4)$alkyl;
$R_3$ is $(C_3-C_6)$alkenyl, $(C_3-C_6)$alkinyl, hydroxy-$(C_1-C_4)$alkyl, $(C_1-C_4)$alkoxy-$(C_1-C_4)$alkyl, di-$(C_1-C_4)$alkylamino-$(C_1-C_4)$alkyl, cyano-$(C_1-C_4)$alkyl, $(C_1-C_4)$alkylthio-$(C_1-C_4)$alkyl, $(C_5-C_6)$cycloalkyl, methylcyclohexyl, phenyl-$(C_1-C_4)$alkyl, pyridyl or pyrimidyl; or
$R_2$ and $R_3$ together with the adjacent N-atom form a 4 to 7-membered ring in which up to 2 carbon atoms can be replaced by N and/or O, which comprises reacting

a) phenoxy-phenoxy-propionic acid halides of the formula

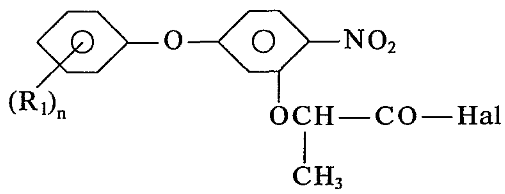

(II)

or phenoxy-phenoxy-propionic acid esters of the formula

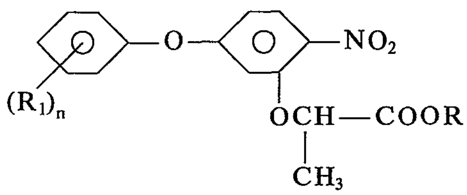

(III)

in which R is $(C_1-C_8)$alkyl, with amines of the formula

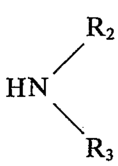

(IV)

or

b) reacting 3-hydroxy-4-nitro-diphenyl ethers of the formula

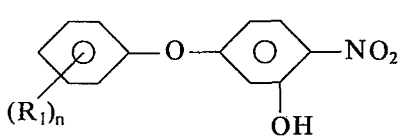

(V)

or correspondings phenolates, with halo-propionic acid amides of the formula

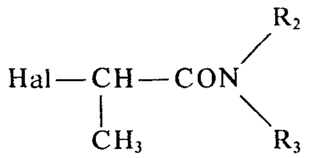

(VI)

in which Hal is chlorine or bromine, optionally in the presence of acid-binding agents.

2. Herbicidal agents, characterized by a content of a compound of the formula I.
3. The use of compounds of formula I for the combatting of weeds.

**Revendications pour les Etats contractants: BE, CH, DE, FR, GB, IT, NL**

1. Phénoxy-phénoxy-propionamides répondant à la formule générale I

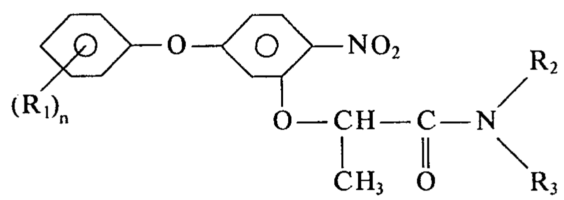

(I)

dans laquelle

$R_1$ représente un halogène, $NO_2$, CN et/ou $CF_3$,
n est égal à 1 ou à 2,
$R_2$ représente l'hydrogène ou un alkyle en $C_1-C_4$,
$R_3$ représente un alcényle en $C_3-C_6$, un alcynyle en $C_3-C_6$, un hydroxyalkyle en $C_1-C_4$, un $(C_1-C_4)$alcoxy-$(C_1-C_4)$alkyle, un di-$(C_1-C_4)$alkylamino-$(C_1-C_4)$alkyle, un cyano-$(C_1-C_4)$alkyle, un $(C_1-C_4)$alkylthio-$(C_1-C_4)$alkyle, un cycloalkyle en $C_5$ ou $C_6$, un méthyl-cyclohexyle, un phényl-$(C_1-C_4)$alkyle, un radical de pyridine ou un radical de pyrimidine, ou encore
$R_2$ et $R_3$ forment ensemble et avec l'atome d'azote voisin un cycle qui contient de 4 à 7 maillons et dans lequel un ou deux atomes de carbone peuvent être remplacés par N et/ou O.

2. Procédé de préparation de composés de formule générale I, procédé caractérisé en ce que:

a) on fait réagir des halogénures d'acides phénoxy-phénoxy-propioniques de formule:

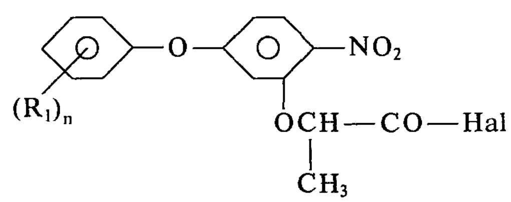

(II)

ou des esters d'acides phénoxy-phénoxy-propioniques de formule

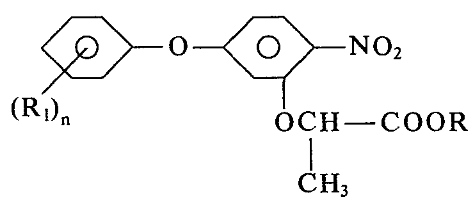

(III)

(où R représente un alkyle en $C_1$—$C_8$), avec des amines de formule

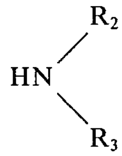 (IV)

ou

b) on fait réagir des phénoxy-1 hydroxy-3 nitro-4 benzènes de formule

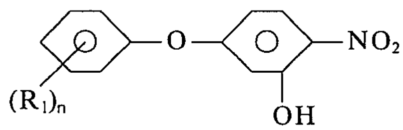 (V)

ou des phénolates correspondants, avec des halogéno-propionamides répondant à la formule

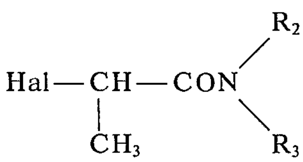 (VI)

dans laquelle »Hal« représente le chlore ou le brome, éventuellement en présence d'accepteurs d'acides.

3. Produits herbicides caractérisés en ce qu'ils contiennent un composé de formule I.
4. Application de composés de formule I à la lutte contre des plantes nuisibles.
5. Hydroxy-éthyl-amide de l'acide [(dichloro-2,4 phénoxy)-3 nitro-6 phénoxy]-2 propionique.
6. Méthoxy-éthylamide de l'acide [(chloro-2 trifluorométhyl-4 phénoxy)-3 nitro-6 phénoxy]-2 propionique.
7. Méthylthio-éthylamide de l'acide [(chloro-2 trifluorométhyl-4 phénoxy)-3 nitro-6 phénoxy]-2 propionique.
8. Hydroxyéthylamide de l'acide [(chloro-2 trifluorométhyl-4 phénoxy)-3 nitro-6 phénoxy]-2 propionique.

**Revendications pour l'Etat contractant: AT**

1. Procédé de préparation de phénoxy-phénoxy-propionamide répondant à la formule générale I

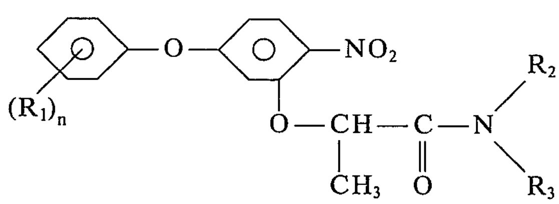 (I)

dans laquelle

$R_1$ représente un halogène, $NO_2$, CN et/ou $CF_3$,
n est égal à 1 ou à 2,
$R_2$ représente l'hydrogène ou un alkyle en $C_1-C_4$,
$R_3$ représente un alcényle en $C_3-C_6$, un alcynyle en $C_3-C_6$, un hydroxyalkyle en $C_1-C_4$, un $(C_1-C_4)$alcoxy-$(C_1-C_4)$alkyle, un di-$(C_1-C_4)$alkylamino-$(C_1-C_4)$alkyle, un cyano-$(C_1-C_4)$alkyle, un $(C_1-C_4)$alkyl-thio-$(C_1-C_4)$alkyle, un cycloalkyle en $C_5$ ou $C_6$, un méthyl-cyclohexyle, un phényl-$(C_1-C_4)$alkyle, un radical de pyridine ou un radical de pyrimidine, ou encore
$R_2$ et $R_3$ forment ensemble et avec l'atome d'azote voisin un cycle qui contient de 4 à 7 maillons et dans lequel un ou deux atomes de carbone peuvent être remplacés par N et/ou O,

procédé caractérisé en ce que:

a) on fait réagir des halogénures d'acides phénoxy-phénoxy-propioniques de formule:

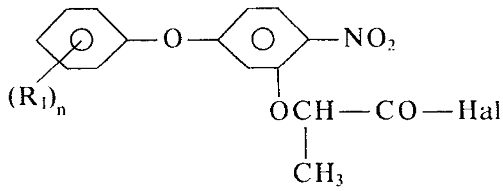

(II)

ou des esters d'acides phénoxy-phénoxy-propionique de formule

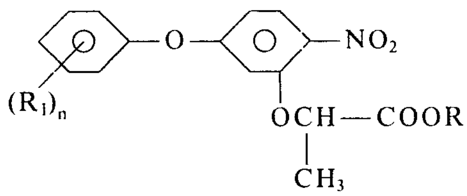

(III)

(où R représente un alkyle en $C_1$—$C_8$), avec des amines de formule

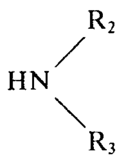

(IV)

ou

b) on fait réagir des phénoxy-1 hydroxy-3 nitro-4 benzènes de formule

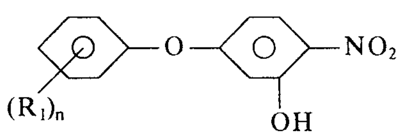

(V)

ou des phénolates correspondants, avec des halogéno-propionamides répondant à la formule

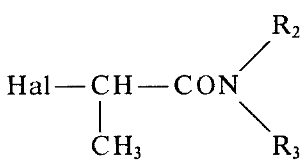

(VI)

dans laquelle »Hal« représente le chlore ou le brome, éventuellement en présence d'accepteurs d'acides.

2. Produits herbicides caractérisés en ce qu'ils contiennent un composé de formule I.
3. Application de composés de formule I pour combattre des plantes nuisibles.